(19) Europäisches Patentamt
European Patent Office
Office européen des brevets

(11) EP 1 786 322 B1

(12) **EUROPEAN PATENT SPECIFICATION**

(45) Date of publication and mention of the grant of the patent:
**28.10.2009 Bulletin 2009/44**

(21) Application number: **05755895.9**

(22) Date of filing: **20.06.2005**

(51) Int Cl.:
***A61B 5/053*** *(2006.01)* ***G01R 33/02*** *(2006.01)*

(86) International application number:
**PCT/EE2005/000008**

(87) International publication number:
**WO 2005/122889 (29.12.2005 Gazette 2005/52)**

(54) **SIMULTANEOUS DISCRETE-TIME ANALYSIS OF FEATURES OF SUBSTANCES**

SIMULTANE ZEITDISKRETE ANALYSE VON MERKMALEN EINER SUBSTANZ

ANALYSE SIMULTANEE DISCRETE TEMPORELLE DES CARACTERISTIQUES D'UNE SUBSTANCE

(84) Designated Contracting States:
**AT BE BG CH CY CZ DE DK EE ES FI FR GB GR HU IE IS IT LI LT LU MC NL PL PT RO SE SI SK TR**

(30) Priority: **18.06.2004 US 580831 P**

(43) Date of publication of application:
**23.05.2007 Bulletin 2007/21**

(73) Proprietor: **Tallinn University of Technology**
**19086 Tallinn (EE)**

(72) Inventors:
- **MIN, Mart**
  **EE13424 Tallinn (EE)**
- **LAND, Raul**
  **EE12611 Tallinn (EE)**
- **PARVE, Toomas**
  **EE10917 Tallinn (EE)**
- **MÄRTENS, Olev**
  **EE13415 Tallinn (EE)**
- **RONK, Ants**
  **EE10611 Tallinn (EE)**

(74) Representative: **Koppel, Mart Enn et al**
**Kajaka Str. 4,**
**Suite 10**
**11317 Tallinn (EE)**

(56) References cited:
**WO-A-2004/047635**
**US-B1- 6 236 951**
**US-B1- 6 494 832**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 786 322 B1

## Description

BACKGROUND OF THE INVENTION

TECHNICAL FIELD

The invention relates to the field of methods for simultaneous discrete-time analysis of features of systems and substances, and more precisely, to methods of measuring electrical impedance, including electrical bioimpedance of such systems and substances (e.g., biological tissue). The method involves simultaneous multifrequency measurement using synchronous sampling.

BACKGROUND ART

Biomedical background

Measurement of electrical bioimpedance (EBI) enables to characterize conditions of a tissue, to get diagnostic images, and to find hemodynamical parameters. An excitation current is applied to the tissue under the study and a voltage response is measured. There are two different current paths through the tissue, the first one proceeds through the extracellular fluid and has a resistive character, and the other (intracellular) passes through the cell membranes and thus, has a capacitive character (see, e.g., M. Min, S. Ollmar, E. Gersing, "Electrical impedance and cardiac monitoring -Technology, potential and applications," International Journal of Bioelectromagnetism, vol. 5, No. 1, pp. 53-56, 2003).

The electrical bioimpedance $\dot{Z}=V/I_{exc}$ can be expressed as a complex parameter.

$$\dot{Z} = R + jX = Z \cdot e^{j\Phi} \qquad (1)$$

with a real part R and an imaginary (capacitive) part X, or a magnitude Z and a phase $\Phi$.

Engineering background

As a rule, parameterization of different compartments of a tissue or an organ is required. Therefore, several (multiple) electrodes and multichannel instruments are used. Traditionally, in multichannel measurements, both the excitation and response signals both are multiplexed. This solution has at least two substantial drawbacks. First, serial multiplexing causes a time delay between measurements and also transient processes in the tissue and measuring instrument. Second, switching during multiplexing induces some additional electrical charges into the tissue and therefore distorts the excitation. As a result, significant uncertainties corrupt the measurement results.

Furthermore, traditionally tunable bandpass filters are used to separate response signals with different frequencies from each other, see e.g., US Patent 5282840 to Hudrlik, patent granted on 1 February 1994, but it makes changing (tuning) excitation frequencies cumbersome and unstable. As a result, an additional likelihood of corruption of measurement information (especially of phase information) appears.

Another method for simultaneous multifrequency measurement uses narrow pulses for excitation, applying analog or digital filtering (see US Patent 5454377 to Dzwonczyk, patent granted on 3 October 1995) for separating different frequency components of the response signal (see M.Howie et al, "An evaluation of a new two-electrode myocardial electrical impedance monitor for detecting myocardial ischemia", Anesth Analg 2001; 92: 12-18). The drawback of this method is that the excitation power is spread over a wide frequency spectrum, and therefore, the excitation at certain predetermined frequency is weak and it is hard to recover the useful signal components from a wideband noise floor. Also, the phase information is not reliable in this case.

A PCT patent application WO 2004/047635, inventor Kennedy, published on 10 June 2004, describes a method of measuring bioimpedances at several predetermined frequencies simultaneously, and using a modern digital signal processing, preferably Fast Fourier Transform (FFT) is proposed for extraction of the response signals with different frequencies. Similar methods have been known for decades and have been used in stationary laboratory systems, but applications in wearable and implantable devices have been restricted due to their complexity and power consumption. Still, as a result of fast developments in microelectronics and computer technology over last few years, these methods could already be implemented in modern wearable medical devices.

However, transforming the time domain processes into frequency domain and applying then FFT for frequency domain extraction to different frequency components from the composite response signal, and applying the inverse FFT for getting back the time domain processes, is a complicated digital processing which requires powerful processors for performing it in real time and fulfilling the Nyquist criterion (sampling rate must be at least two to five time of the frequency (see page 9 of the published application).

In practice, portable, wearable, and implantable devices need specialized, faster, simpler, and less energy consuming digital processors (in comparison with the commercially available ones) and more specialized fast signal processing algorithms (in comparison with FFT) for solving the task of parallel (simultaneous) processing of multiple different frequency signals in real time. The clock frequency of the processor and data flow through the processor must be minimized using the simplest algorithms and minimal sampling rates.

Therefore, new method for digital signal processing is proposed, using undersampling at much lower than the Nyquist's rate (i.e., less than 2 times the frequency of the sampled signal).

DISCLOSURE OF THE INVENTION

One aspect of the invention is a method for simultaneous discrete time analysis of features of systems or substrates by undersampling multifrequency signals below Nyquist's rate. The method is used for measuring bioimpedance with a set of excitation signals, applied to an object, such as biological tissue, simultaneously and extracting each component with different frequencies from the response signal by sampling the response signal with a set of suitably selected pulses, whereas the sampling rate is well below Nyquist rate.

Disclosed is also a multichannel bioimpedance analyzer, which applies the above method.

The method is suitable for monitoring patients heart and/or lungs and his/her whole cardiovascular and pulmonary systems, e.g., during and after heart surgery operation performing MIMO (multiple-input-multiple-output) bioimpedance measurement without multiplexing of excitation and without filtering the response to the excitation. Method is also suitable for application in wearable and implantable cardiac monitors, pacemakers and defibrillators, as well as other situations of medical monitoring and diagnosing where bioimpedance based information can be used.

Method according to one aspect of the invention comprises the steps of generating a set of excitation signals with predetermined frequencies and applying said set of excitation signals simultaneously to an object, receiving a multicarrier response signal from said object, generating a set of sampling pulses for a predetermined observation time slot; demodulating said response signal by sampling said signal using said set of sampling pulses, wherein said sampling is performed for each carrier of said response signal simultaneously; and obtaining a set of main frequency tagged estimates, each of said estimates comprising a plurality of sub-estimates, said sub-estimates representing a signal value, taken for said observation time slot.

The multichannel and multifrequency method can used for measurement of electrical bioimpedance 1) of several different compartments of the organ (e.g. heart and lungs) or tissue at the same frequency (in fact, the measurements are performed at slightly different frequencies, see Fig. 1); and 2) of the same compartment at several frequencies which differ substantially (for performing frequency response analysis or spectroscopy, as in Fig. 2).

The devices must perform the bioimpedance analysis in real time and has to process several dynamic variations simultaneously.

BRIEF DESCRIPTION OF THE DRAWINGS

Fig. 1 depicts simultaneous two frequency measurement of impedance at slightly different frequencies ($f_2$ is approximately 0.9 $f_1$) using spatially shifted electrodes (measurement of different compartments of a tissue), where $V_{rsp}$ is a response signal.

Fig. 2 depicts simultaneous two frequency measurement of impedance at substantially different frequencies ($f_2 \ll f_1$) using a single input electrode (spectroscopy of a single compartment of a tissue).

Fig. 3 depicts a block diagram of a multicarrier and multichannel bioimpedance analyzer according to one embodiment of the present invention.

Fig. 4 depicts a block diagram of a sine wave synthesizer of multifrequency excitation signal, and a synthesizer of related sampling pulses.

Fig. 5 depicts a paraphase quadrature undersampling of a sine wave signal at a phase shift of 30 degrees and a train of corresponding sampling pulses, where samples are sorted into inphase (+I = Re+ , and -I = Re-) and quadrature (+Q = Im+ , and -Q = Im-) components, and where

$$Re = (Re^+ - Re^-)/2, Im = (Im^+ - Im^-)/2,$$

and

$$\text{offset DC} = (Re^+ + Re^-)/2 = (Im^+ + Im^-)/2$$

Fig. 6 depicts two excitation signals with slightly different frequencies ($f_1$ / $f_2$ = 6/5) within one observation time slot, and corresponding non-uniform trains of sampling pulses with formation of estimates for amplitudes $A_1$ and $A_2$ of the both excitations with frequencies $f_1$ and $f_2$.

Fig. 7 illustrates the handling and processing of sampled data with an analyzer according to one embodiment of the invention.

MODES FOR CARRYING OUT THE INVENTION

The multichannel and multifrequency method can used for measurement of electrical bioimpedance

1) of several different compartments of the organ (e.g. heart and lungs) or tissue at the same frequency (in fact, the measurements are performed at slightly different frequencies, see Fig. 1);
2) of the same compartment at several frequencies which differ substantially (for performing frequency response analysis or spectroscopy, as in Fig. 2).

The configuration in Fig. 1 means that we want to measure two different compartments of the organ at the same time and at the same frequency. For that purpose, we actually use two slightly different frequencies to separate the responses at the same output. Frequency difference must be negligible for the frequency response of tissue impedance.

The configuration in Fig. 2 describes the case when we want to measure the same compartment of the organ at the principally different frequencies at the same time. That is, we want to study the frequency response of parameters of the tissue impedance, i.e. to perform dynamic impedance spectroscopy. Dynamics means a variation of impedance parameters due to ongoing physiological processes (heart beating, breathing, other muscular activity).

Frequencies of the simultaneously applied sinusoidal excitations vary simultaneously in a large range (e.g. from 1 kHz up to 10 MHz, typically). The main idea of the proposed approach is that the use of a rather specific signal system (frequencies of sinusoidal excitations are carefully selected and sampling pulses are properly related/adapted to excitation frequencies) makes it possible to separate responses to different excitations from the measured summary signals by means of synchronous sampling at different sampling rates. As a result, fast and simple signal processing algorithms and simple signal processor architectures can be implemented.

Disclosed is a novel solution of the multichannel bioimpedance analyzer, which enables

1) to generate and send simultaneously (without multiplexing) through several k excitation electrodes k sine wave excitation signals of different frequencies, which form a set $f_{SW,k}$, k=1, 2, 3, 4, 5 etc, covering so a wide frequency range;
2) to generate sampling pulses with frequencies $f_{SP,k}$, which are tagged to excitation frequencies so that synchronous undersampling can be applied for every response (carrier) signal;
3) to process the composite summary response signal so that:

   a) the different bioimpedance-modulated responses (carriers) at all the excitation frequencies $f_{SW,k}$, k=1, 2, 4, 5 etc can be extracted, demodulated and identified simultaneously but separately in real time,
   b) a slowly varying additive component ("direct current") of the response signal (caused by bioelectrical activity of the tissue, e.g. ECG, EMG, electronic offset, drift, noise) can be also extracted or cancelled.

One should appreciate that even though in Figs. 1 and 2 there are given only two frequencies (k=2), in principle much more frequencies can be used simultaneously, e.g., up to eight and even more.

First we describe the architecture of the analyzer. Then we describe a signal synthesizer system, which provides the excitation signals and sample trains, which are strictly related to each other. Finally, we present the basics of sampling and a simple signal processing method derived just from/for the used frequency relations.

A block-diagram of the impedance analyzer, where the excitation signals of different frequencies are applied to the tissue without multiplexing is given in Fig 3.

The analyzer contains a source of excitation signals 1 for generating a set of simultaneous excitation signals, at different frequencies, and a sampler 2 for generating sampling pulses synchronized with the excitation signals so that different trains of pulses are tagged to the excitation signals with different frequencies. A multifrequency response to the different excitation signals from an object 3 under study (a bio-object, such as a tissue) are directed through a channel selector 4 to a digital signal analyzer 5, where the multifrequency response signal is sampled simultaneously and synchronously to excitation frequencies during digitizing.

Fig. 4 explains the method of generating (synthesizing) the multiple sine wave excitations and the synchronous sampling pulses corresponding to the each sine wave. The source of excitation signals 1 (Fig. 3) contains a phase locked loop (PLL) 11 (see Fig. 4) for multiplying the clock frequency $f_{clk}$ by N. The multiplied frequency $f_{clk}$*N is applied to a sine wave synthesizer 12 of excitation signals and to a sampling pulse synthesizer 13. Synthesized sine waves and sampling pulses are synchronized in this way for obtaining synchronous sampling or undersampling (sampling rate is lower than requires the Nyquist rate of response signals). The excitation signals result in the response voltage as the sum of individual excitations. Sampling pulses conjugate as the single non-uniform train of pulses. In Fig. 4, a letter Σ marks summing of individual sine waves into one composite excitation in the biological object, and OR denotes a logical conjunction of individual sampling pulses into one sampling pulse train.

The sampler determines sampling instances so that they ensure simultaneous and synchronous digitizing of the response to all the used different frequency excitation signals in order to evaluate complex impedance of different compartments of the tissue or to perform a spectral analysis (spectroscopy) simultaneously. As responses to different excitations are of interest, the digitized summary response is decomposed into these components applying appropriate data processing in the digital signal analyzer 5. The samples of the separated components are used to calculate a real part Re (resistive component R) and an imaginary part Im (capacitive component X) as well as magnitude Z and phase Φ of every complex bioimpedance Z measured at different frequencies.

Sampling of a single sine wave response signal to determine its real (Re) and imaginary (Im) parts or inphase I and quadrature Q components using synchronous uniform undersampling is given in Fig. 5, where the inphase and quadrature sampling pulses are shifted by 90˚. Additional paraphase pulses shifted by 180' are used to determine $Re^-$ and $Im^-$ values for extracting an additive signal component or slowly changing DC signal (offset) through the data $Re^+$ and $Re^-$ or $Im^+$ and $Im^-$, where

$$DC = (Re^+ + Re^-)/2 = (Im^+ + Im^-)/2. \qquad (2)$$

The real and imaginary parts or the inphase and quadrature components can be calculated as simply as

$$Re = (Re^+ - Re^-)/2, \text{ and } Im = (Im^+ - Im^-)/2 \qquad (3)$$

Synchronous undersampling of a single response signal (sampling rate is lower than signal frequency, i.e. Nyquist criterion is not fulfilled) to measure its real (Re - a dot) and imaginary (Im - a square) parts, and to separate an additive component (offset), using sorting and summing of data (see Fig. 7) is given in Fig. 5 graphically.

Fig 6 describes the sampling of two response signals with slightly different relative frequencies $f_1 = 6/5 \times f_2$ and estimation of amplitudes of these signals. Into one observation time slot there are placed 5 periods of the frequency $f_1$ and 6 periods of $f_2$. The rule is that within one observation (measurement) time slot there must be a space for integer number of periods of each response signals to be measured and analyzed.

It can be shown that when the inphase undersampling (two times lower than the Nyquist criterion requires) at the frequency $f_1$ (dots) is performed, then the mean value of samples of the first signal (the sine wave with the frequency $f_1$) gives the amplitude value $A_1$ of this signal, but the sum of samples of the other signal (the sine wave with the frequency $f_2$) gives zero. It means that only the first signal is measured in this case (the second signal is cancelled or zeroed out).

When the inphase sampling at the the frequency $f_2$ (squares) is performed, then the sum of samples of the first signal (the sine wave with the frequency $f_1$) gives the zero result, but the mean value of samples of the other signal (the sine wave with the frequency $f_2$) gives the amplitude value $A_2$ of this signal. It means that only the second signal is measured in this case (the first signal is cancelled or zeroed out).

In this way, using synchronised non-uniform sampling (preferrably undersampling) and a special handling of sampled data, we can obtain the parameters of responses to different excitations separately and we can cancel also additive components escorting this signal, e.g. low frequency noise, DC offset or even extract the useful additive ECG or EMG signals.

Mathematical formulation of calculations is given as follows. Data handling is described in Fig. 7.

Summing up the sorted data at the sampling rate $f_{SP,1}$ within one observation time slot gives as

$$A_1 = \sum_{i=1}^{6} D(f_1)/6 = 1.0 \qquad (4)$$

$$\sum_{j=1}^{5} D(f_2)/6 = 0 \qquad (5)$$

and summing up the samples obtained at the sampling rate rate $f_{SP,2}$ within one observation time slot gives

$$A_2 = \sum_{j=1}^{5} D(f_2)/5 = 1.0 \qquad (6)$$

$$\sum_{i=1}^{6} D(f_1)/5 = 0 \qquad (7)$$

Data handling and signal processing are given in Fig. 7, where samples of signals digitized in the analog-to-digital converter (ADC) 51 will be sorted (see a sorter 52) by their carrier frequencies. The sorted data are multiplexed by digital multiplexers 53 and 54 into respective memory registers (55 to 58, and 59 to 62 in Fig. 7), and will be processed in a digital signal processor 63 by algorithms, which follow equations (2) to (7). The results $Re_{fl}$ ... $Re_{fk}$, $Im_{fl}$ ... $Im_{fk}$, and DC are forwarded to the output 64.

Advantages of the invention

The invention has several advantages. The same hardware can be used for software based generating of several instruments for different heart and lung monitoring goals (rate and timing, hemodynamic parameters, edema, ischemia), e.g., during and after the open chest surgery.

The method enables to follow the bioimpedance changes in several compartments of heart and lungs at the same time and at several frequencies simultaneously. Synchronous undersampling reduces the data flow and required computing power to minimum, lowering so the energy need to a battery level.

Simple signal processing algorithms, which contains only addressing, sorting and summing up procedures without any closed loop processing cycles ensures maximal speed of digital processing.

Thanks to simplicity and low power consumption, the cheap wearable and implantable devices can be designed using the invented method.

The exemplary embodiments presented herein illustrate the principles of the invention and are not intended to be exhaustive or to limit the invention to the form disclosed; it is intended that the scope of the invention be defined by the claims appended hereto and their equivalents.

## Claims

**1.** Method for simultaneous discrete-time analysis of features of systems and substances, comprising
generating a set of excitation signals with predetermined frequencies and applying said set of excitation signals simultaneously to an object,
receiving a multicarrier response signal from said object, said response signal being modulated in said object; **characterized in that**:
generating a set of sampling pulses for a predetermined observation time slot;

demodulating said response signal by sampling said signal using said set of sampling pulses, wherein said sampling is performed for each carrier of said response signal simultaneously; and
obtaining a set of main frequency tagged estimates, each of said estimates comprising a plurality of sub-estimates, said sub-estimates representing a signal value, taken for said observation time slot.

2. A method according to claim 1, **characterized in that** said response signal is sampled non-uniformly within said observation time slot.

3. A method according to claim 2, **characterized in that** said observation time slot contains an exact integer number of periods of each carrier frequency, whereas each of said frequency tagged estimates is sorted into a separate adders so that said estimate at each frequency of interest can be obtained and the impact of other estimates is cancelled.

4. A method according to claim 3, **characterized in that** an inphase sampling is applied for said frequency of interest, wherein obtained inphase samples are used to separate inphase estimates.

5. A method according to claim 3***, characterized*** **in that** a quadrature sampling is applied for said frequency of interest, wherein obtained quadrature samples are used to separate quadrature estimates.

6. A method according to claim 3, **characterized in that** a paraphase sampling is applied for each frequency of interest, whereas obtained paraphase samples are used for extracting the additive signal components or removal of the offset and common-mode noise by summing cancellation technique.

7. A method according to claim 1, **characterized in that** an undersampling is applied to demodulate at least one of said carriers of said response signal.

8. A method according to claim 1, **characterized in that** said set of excitation signals is applied dispersedly to said object.

9. A method according to claim 8, **characterized in that** said predetermined frequencies are within about 10% from each other.

10. A method according to claim 1, **characterized in that** said set of excitation signals is applied at a single location to said object.

## Patentansprüche

1. Verfahren zur zeitdiskreten Simultananalyse der Charakteristiken von Systemen und Substanzen, einschließlich der Generierung einer Reihe von Anregungssignalen mit vorbestimmten Frequenzen und der simultanen Anwendung der Reihe von Anregungssignalen auf ein Objekt, wobei von diesem Objekt ein Multiträger-Rückmeldungsssignal erhalten wird und dieses Rückmeldungssignal in diesem Objekt moduliert wird, **dadurch gekennzeichnet, dass**: für das vorbestimmte Beobachtungszeitfenster eine Reihe von Abtastimpulsen generiert wird; das Rückmeldungssignal durch Abtastung des Signals unter Anwendung einer Reihe von Abtastimpulsen demoduliert wird, wobei das Abtasten für jeden Träger des Rückmeldungssignals simultan ausgeführt wird; und eine Reihe von frequenzmarkierten Schätzwerten erhalten wird, wobei jeder dieser Schätzwerte eine Vielzahl von Teilschätzwerten beinhaltet und diese Teilschätzwerte einen Signalwert für das Beobachtungszeitfenster repräsentieren.

2. Verfahren nach Anspruch 1, **dadurch gekennzeichnet, dass** das Rückmeldungssignal innerhalb des Beobachtungszeitfensters uneinheitlich abgetastet wird.

3. Verfahren nach Anspruch 2, **dadurch gekennzeichnet, dass** das Beobachtungszeitfenster eine genaue Ganzzahl der Perioden jeder Trägerfrequenz beinhaltet, wobei jeder der frequenzmarkierten Schätzwerte in separate Addierer sortiert wird, so dass bei jeder das Interesse weckenden Frequenz ein Schätzwert erhalten werden kann und die Auswirkung anderer Schätzwerte gelöscht wird.

4. Verfahren nach Anspruch 3, **dadurch gekennzeichnet, dass** auf die das Interesse weckende Frequenz das phasenrichtige Abtasten angewandt wird, wobei die erhaltenen phasenrichtigen Abtastungen zur Trennung der phasenrichtigen Schätzwerte verwendet werden.

5. Verfahren nach Anspruch 3, **dadurch gekennzeichnet, dass** auf die das Interesse weckende Frequenz das Quadraturabtasten angewandt wird, wobei die erhaltenen Quadraturabtastungen zur Trennung Separierung der Quadraturschätzwerte verwendet werden.

6. Verfahren nach Anspruch 3, **dadurch gekennzeichnet, dass** auf jede das Interesse erweckende Frequenz das gegenphasige Abtasten angewandt wird, wobei die erhaltenen gegenphasigen Abtastungen zur Extraktion der zusätzlichen Signalkomponenten oder zur Beseitigung des Offset- und Gleichtaktstörgeräushes durch Aufsummierung der Löschtechnik verwendet werden.

7. Verfahren nach Anspruch 1, **dadurch gekennzeichnet, dass** zur Demodulation von mindestens einem Träger des Rückmeldungssignals die Unterabtastung verwendet wird.

8. Verfahren nach Anspruch 1, **dadurch gekennzeichnet, dass** eine Reihe von Anregungssignalen zerstreut auf das Objekt angewandt wird.

9. Verfahren nach Anspruch 8, **dadurch gekennzeichnet, dass** die vorbestimmten Frequenzen etwa innerhalb von 10% voneinander liegen.

10. Verfahren nach Anspruch 1, **dadurch gekennzeichnet, dass** die Reihe von Anregungssignalen an einem einzelnen Ort auf das Objekt angewandt wird.

## Revendications

1. Méthode pour l'analyse simultanée en temps discret des caractéristiques de systèmes et substances, comprenant la génération d'un ensemble de signaux d'excitation avec des fréquences prédéfinies et l'application dudit ensemble de signaux simultanément sur un objet, la réception d'un signal de réponse multi-porteur depuis ledit objet, ledit signal de réponse étant modulé dans ledit objet; **caractérisée en**: la génération d'un ensemble d'impulsions d'échantillonnage pour un intervalle de temps d'observation prédéfini ; la démodulation dudit signal de réponse en échantillonnant ledit signal en utilisant ledit ensemble d'impulsions d'échantillonnage, en quoi ledit échantillonnage est réalisé pour chaque porteur dudit signal de réponse simultanément ; et l'obtention d'un ensemble d'estimations marquées de la fréquence principale, chaque dite estimation comprenant une pluralité de sous-estimations, lesdites sous-estimations représentant une valeur de signal, prise pour ledit intervalle de temps d'observation.

2. Une méthode selon la revendication 1, **caractérisée en ce que** ledit signal de réponse est échantillonner non uniformément durant ledit intervalle de temps d'observation.

3. Une méthode selon la revendication 2, **caractérisée en ce que** ledit intervalle de temps d'observation contient un nombre entier de périodes pour chaque fréquence porteuse, alors que chaque estimation marquée de fréquence est triée dans une section de mélange séparée de sorte que ladite estimation de chaque fréquence d'intérêt peut être obtenue et l'impact des autres estimations est annulé.

4. Une méthode selon la revendication 3, **caractérisée en ce qu'**un échantillonnage en phase est appliqué pour ladite fréquence d'intérêt, en quoi les échantillons en phase obtenus sont utilisés pour séparer les estimations en phase.

5. Une méthode selon la revendication 3, **caractérisée en ce qu'**un échantillonnage en quadrature est appliqué pour ladite fréquence d'intérêt, en quoi les échantillons en quadrature sont utilisés pour séparer les estimations en quadrature.

6. Une méthode selon la revendication 3, **caractérisée en ce qu'**un échantillonnage en déphasage est appliqué pour chaque fréquence d'intérêt, alors que les échantillons en déphasage obtenus sont utilisés pour extraire les composants de signal additif ou pour supprimer le décalage et le bruit en mode commun par la technique d'interférence.

7. Une méthode selon la revendication 1, **caractérisée en ce qu'**un sous-échantillonnage est appliqué pour démoduler au moins un desdits porteurs dudit signal de réponse.

**8.** Une méthode selon la revendication 1, **caractérisée en ce que** ledit ensemble de signaux d'excitation est appliqué de manière dispersée sur ledit objet.

**9.** Une méthode selon la revendication 8, **caractérisée en ce que** lesdites fréquence prédéfinies sont environ à 10 % l'une de l'autre.

**10.** Une méthode selon la revendication 8, **caractérisée en ce que** ledit ensemble de signaux d'excitation est appliqué à un seul endroit dudit objet.

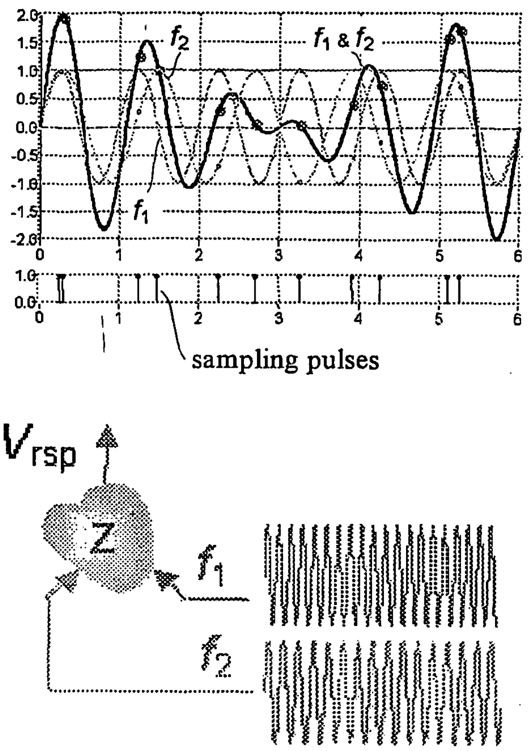
f2
f1 & f2
f1
sampling pulses
Vrsp
Z
f1
f2

Fig. 1

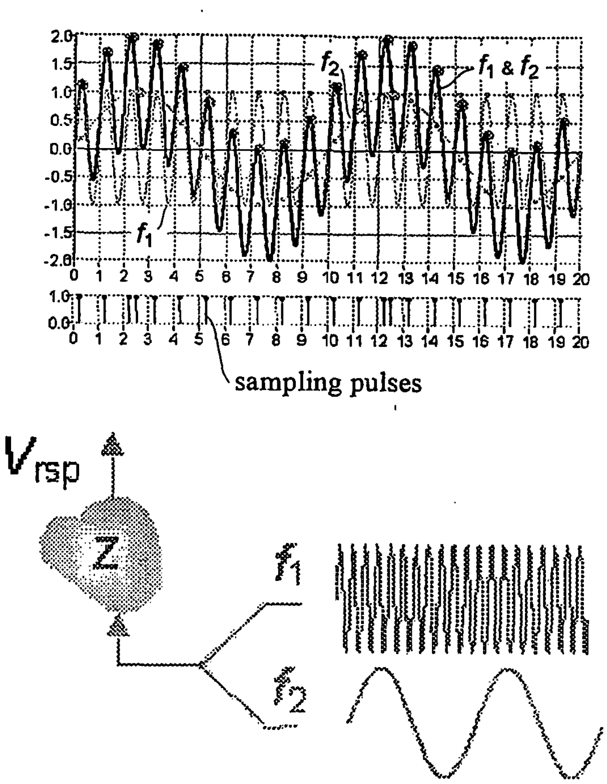
f2
f1 & f2
f1
sampling pulses
Vrsp
Z
f1
f2

Fig. 2

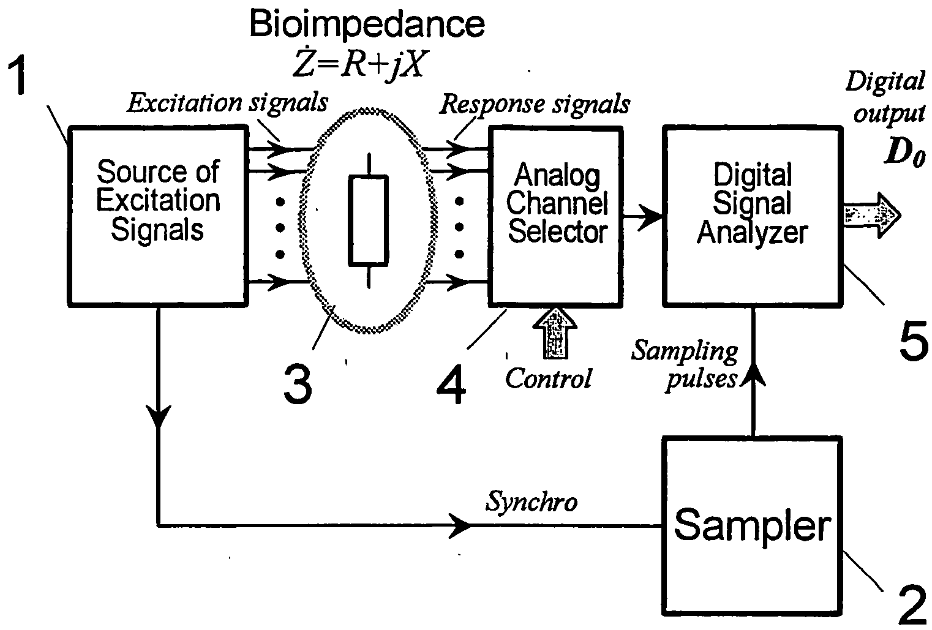
Bioimpedance
$\dot{Z}=R+jX$
1
Excitation signals
Response signals
Digital output
$D_0$
Source of Excitation Signals
Analog Channel Selector
Digital Signal Analyzer
3
4
Control
Sampling pulses
5
Synchro
Sampler
2

Fig. 3

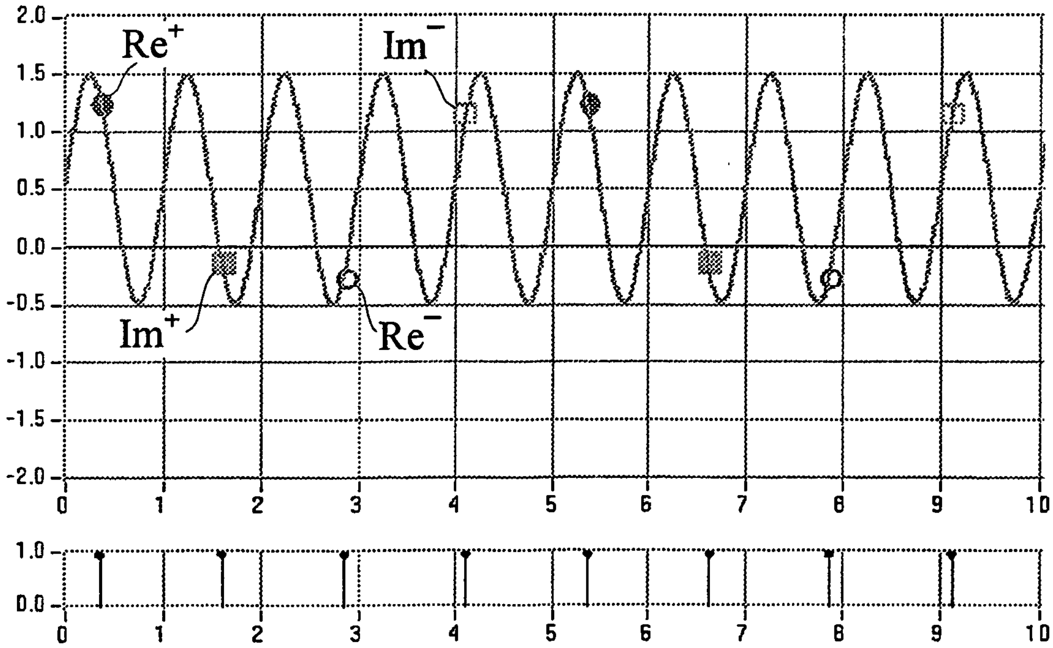
Re+
Im−
Im+
Re−

Fig. 5

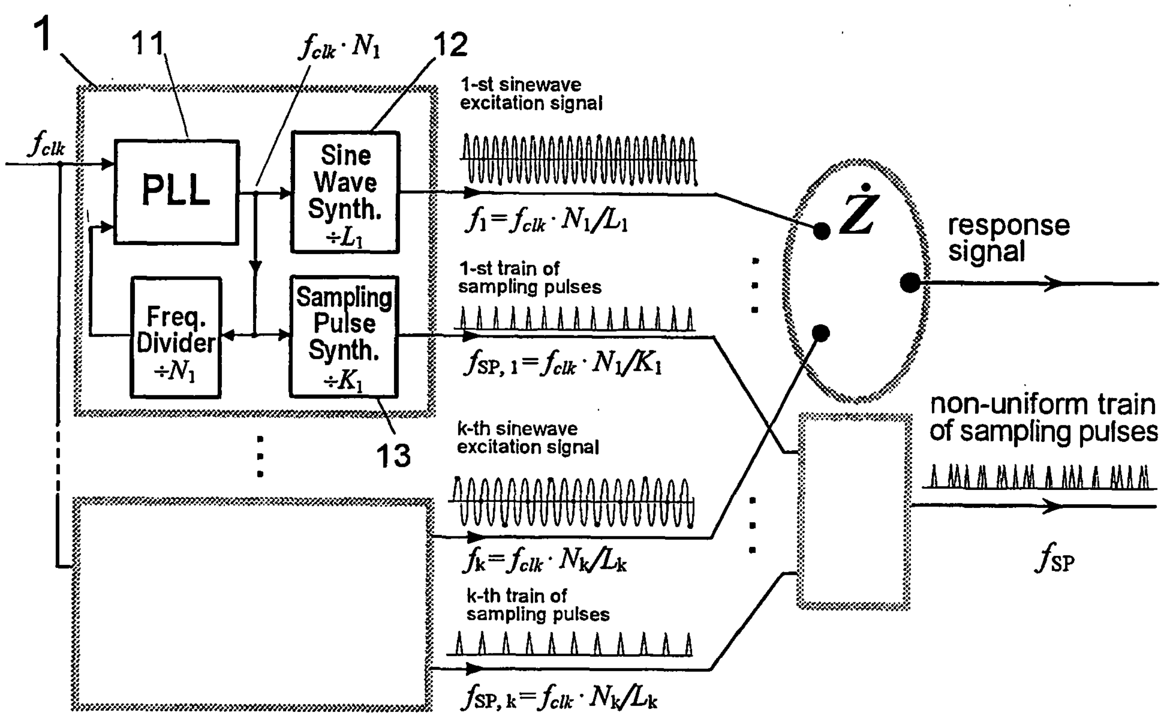
1
11
$f_{clk} \cdot N_1$
12
$f_{clk}$
PLL
Sine Wave Synth. $\div L_1$
Freq. Divider $\div N_1$
Sampling Pulse Synth. $\div K_1$
13
1-st sinewave excitation signal
$f_1 = f_{clk} \cdot N_1/L_1$
1-st train of sampling pulses
$f_{SP,1} = f_{clk} \cdot N_1/K_1$
k-th sinewave excitation signal
$f_k = f_{clk} \cdot N_k/L_k$
k-th train of sampling pulses
$f_{SP,k} = f_{clk} \cdot N_k/L_k$
$\dot{Z}$
response signal
non-uniform train of sampling pulses
$f_{SP}$

Fig. 4

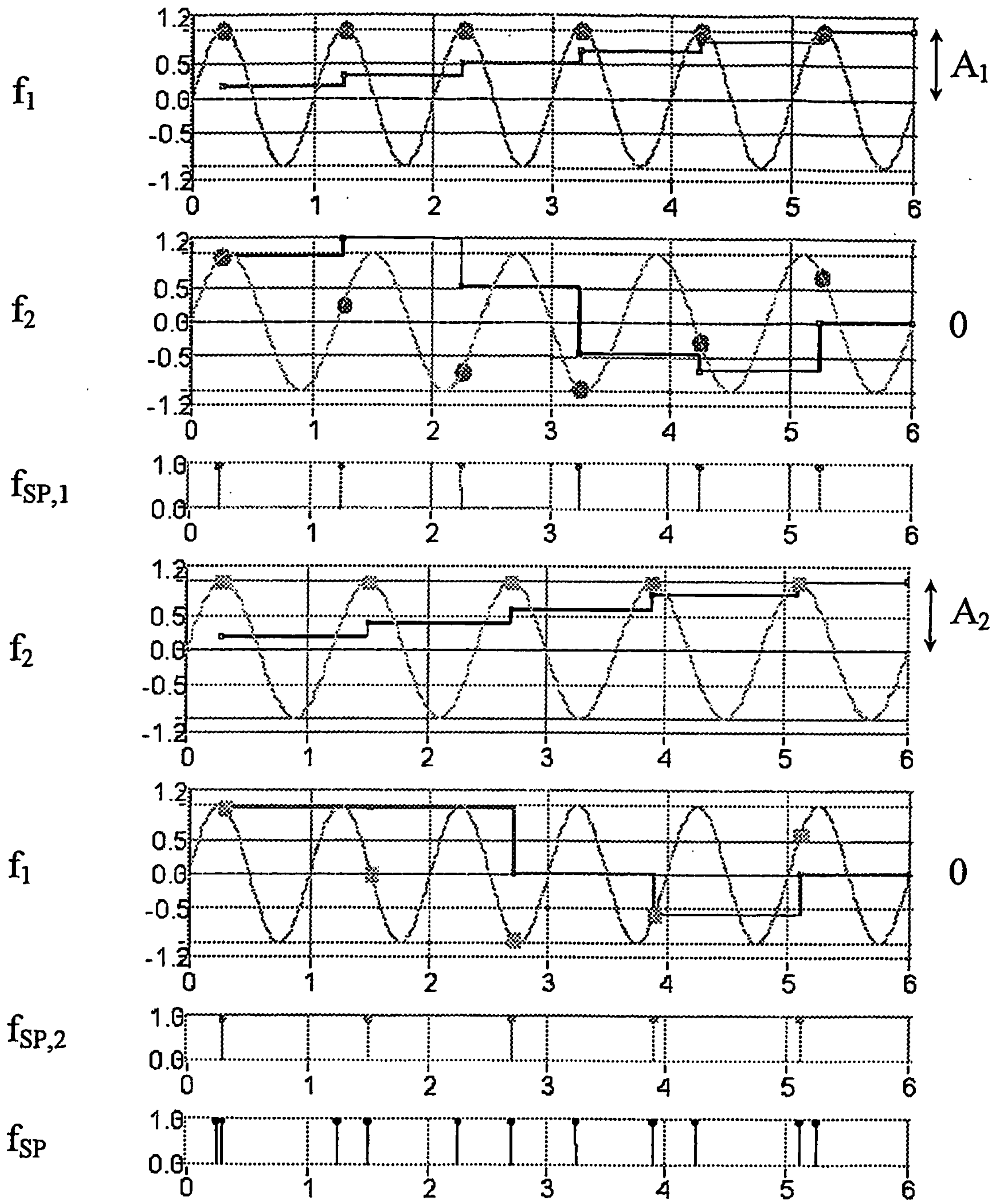


Fig. 6

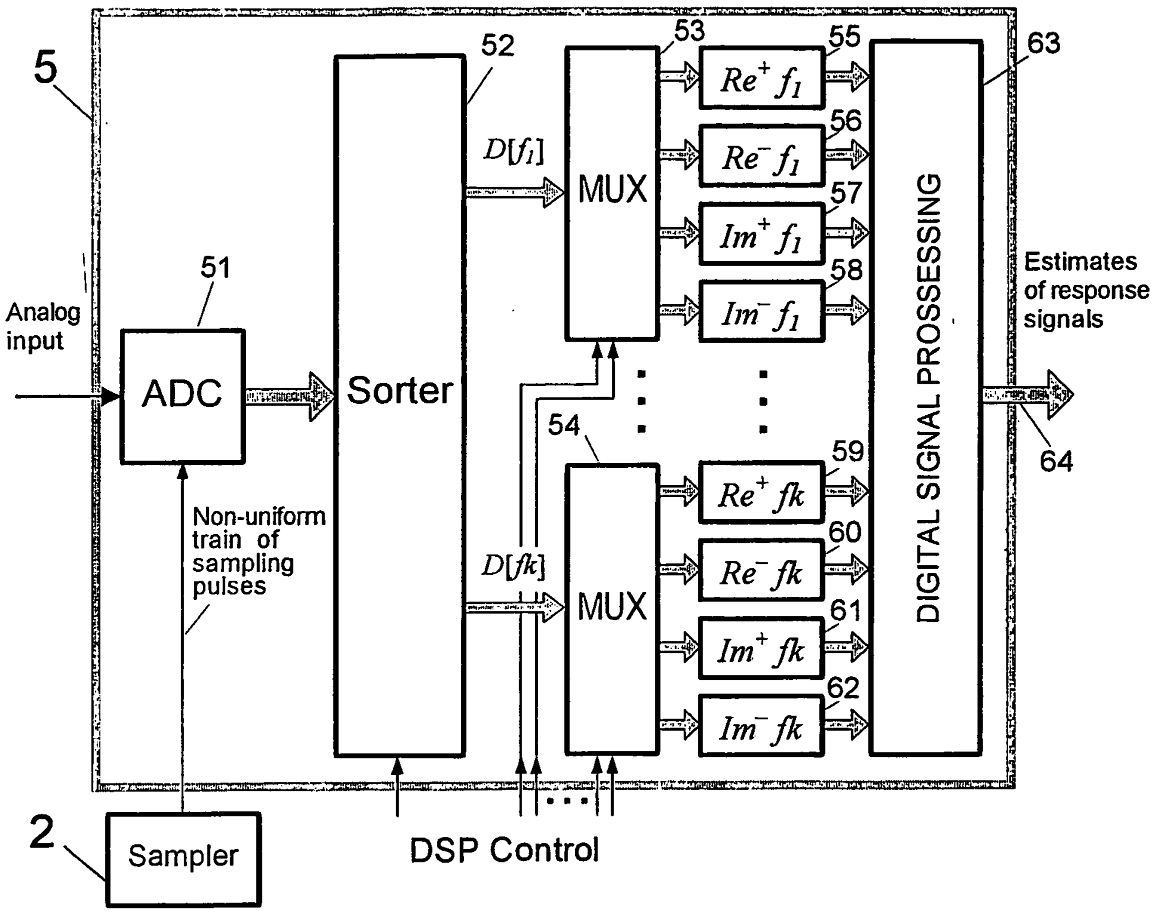
5
Analog input
51
ADC
Non-uniform train of sampling pulses
Sorter
52
$D[f_1]$
MUX
53
54
$D[fk]$
MUX
DSP Control
2
Sampler
$Re^+ f_1$
55
$Re^- f_1$
56
$Im^+ f_1$
57
$Im^- f_1$
58
$Re^+ fk$
59
$Re^- fk$
60
$Im^+ fk$
61
$Im^- fk$
62
DIGITAL SIGNAL PROSSESSING
63
Estimates of response signals
64

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5282840 A, Hudrlik
- US 5454377 A, Dzwonczyk
- WO 2004047635 A, Kennedy


### Non-patent literature cited in the description


- **M. Min ; S. Ollmar ; E. Gersing.** Electrical impedance and cardiac monitoring -Technology, potential and applications. *International Journal of Bioelectromagnetism,* 2003, vol. 5 (1), 53-56
- **M.Howie et al.** An evaluation of a new two-electrode myocardial electrical impedance monitor for detecting myocardial ischemia. *Anesth Analg,* 2001, vol. 92, 12-18